# EUROPEAN PATENT APPLICATION

(11) **EP 1 903 040 A1**
(43) Date of publication of application: **26.03.2008**
(21) Application number: 06780913.7
(22) Date of filing: 04.07.2006
(51) Int. Cl.: C07D 403/12

(54) **METHOD FOR PRODUCING 4(3H)-QUINAZOLINONE DERIVATIVE**

(30) Priority: 05.07.2005 JP 2005196842
(71) Applicant: BANYU PHARMACEUTICAL CO., LTD., Tokyo 102-8667 (JP); Merck & Co., Inc., Rahway, New Jersey 07065-0907 (US)
(72) Inventor: SAWADA, Naotaka, Institute,3, Okuba, Tsukuba-shi ,Ibaraki 300-2611 (JP); TSURITANI, Takayuki, Institute,3, Okuba, Tsukuba-shi, Ibaraki 300-2611 (JP); SATO, Kimihiko, Kanagawa 250-0122 (JP); ITOH, Takahiro, Institute,3, Okuba, Tsukuba-shi, Ibaraki 300-2611 (JP); AKAO, Atsushi, Ibaraki 314-0146 (JP); KADOWAKI, Chie, Institute,3, Okuba, Tsukuba-shi, Ibaraki 300-2611 (JP); IIDA, Takehiko, Institute,3, Okuba, Tsukuba-shi, Ibaraki 300-2611 (JP); YASUDA, Nobuyoshi, Rahway, New Jersey 07065-0907 (US); MASE, Toshiaki, Institute,3, Okuba, Tsukuba-shi, Ibaraki 300-2611 (JP)
(74) Representative: Buchan, Gavin MacNicol
(86) International application number: PCT/JP2006/313639
(87) International publication number: WO 2007/004735

(57) **Abstract**

This invention is related to a method for producing 3-{4-[3-(1-pyrrolidinyl)propoxy]phenyl}-5-trifluoromethyl-4(3H)-quinazolinone comprising a step for reacting 2-methyl-5-trifluoromethyl-4H-3,1-benzoxazin-4-one with 4-[3-(1-pyrrolidinyl)propoxy]aniline or an acid-addition salt thereof, or 4-(1-cyclobutyl-4-piperidiny9oxyaniline or acid addition salt thereof in the presence of an acid catalyst.

## Description

### TECHNICAL FIELD

The present invention relates to a novel, more efficient and safe method for producing a 4(3H)-quinazolinone derivative which is useful as a medicine.

### BACKGROUND ART

A 4(3H)-quinazolinone derivative is useful as a preventive or a remedy for metabolic system diseases such as obesity, diabetes, hormone secretion disorder, hyperlipemia, gout, fatty liver; circulatory system diseases such as stenocardia, acute/congestive heart failure, myocardial infarction, coronary arteriosclerosis, hypertension, renal disease, electrolyte disorder; and central and peripheral nervous system diseases such as bulimia, emotional disorder, depression, anxiety, delirium, dementia, schizophrenia, attention deficit hyperactivity disorder, memory impairment, Alzheimer disease, Parkinson disease, sleep disorder, cognitive disorder, motion disorder, paresthesia, dysosmia, epilepsy, morphine resistance, narcotic dependence, alcoholism (Patent Reference 1). Patent Reference 1 discloses a method for producing a 4(3H)-quinazolinone derivative.

Patent Reference 1: WO2005/077905

### DISCLOSURE OF THE INVENTION

The present invention is to provide a production method excellent for industrial-scale production of a 4(3H)-quinazolinone derivative.

The present inventors have assiduously studied for developing a method for producing a 4(3H)-quinazolinone derivative which is efficient and safe for industrial-scale production of the derivative, and as a result, have found out a production method industrially excellent in the following points (i) to (v) and have completed the present invention.
(i) In a step of producing 2-methyl-3-{4-[3-(1-pyrrolidinyl)propoxy]phenyl}-5-trifluoromethyl-4(3H)-quinazolinone from 2-methyl-5-trifluoromethyl-4H-3,1-benzoxazin-4-one, the yield of the intended compound may be increased.
(ii) A production method has been established in which, in a step of producing an intermediate, 2-amino-6-trifluoromethylbenzoic acid, the yield of the intended compound may be increased.
(iii) A specific salt of 2-amino-6-trifluoromethylbenzoic acid has good crystallinity and may be handled with ease in industrial-scale production.
(iv) A production method has been established in which, in a step of reducing 4-[3-(1-pyrrolidinyl)propoxy]-1-nitrobenzene, a safer reagent can be used.
(v) A production method has been established in which a more inexpensive starting material for 4-[3-(1-pyrrolidinyl)propoxy]aniline can be used.

Specifically, the invention relates to the following (1) to (12):
(1) A method for producing 2-methyl-3-{4-[3-(1-pyrrolidinyl)propoxy]phenyl}-5-trifluoromethyl-4(3H)-quinazolinone, which comprises reacting 2-methyl-5-trifluoromethyl-4H-3,1-benzoxazin-4-one with 4-[3-(1-pyrrolidinyl)propoxy]aniline or an acid-addition salt thereof in the presence of an acetic acid-based catalyst.
(2) The production method of (1), wherein the acid-addition salt of 4-[3-(1-pyrrolidinyl)propoxy]aniline is 4-[3-(1-pyrrolidinyl)propoxy]aniline bis(p-toluenesulfonate).
(3) The production method of (1), wherein the acetic acid-based catalyst is acetic acid.
(4) The production method of (3), wherein the amount of acetic acid is from 10 to 30 times by volume of 4-[3-(1-pyrrolidinyl)propoxy]aniline or the acid-addition salt thereof.
(5) The production method of (1), wherein the acetic acid-based catalyst is acetic acid and sodium acetate.
(6) The production method of (1), wherein the acetic acid-based catalyst is acetic acid, sodium acetate and solvent.
(7) The production method of (6), wherein the solvent is toluene or tetrahydrofuran.
(8) The production method of (1), wherein 2-methyl-5-trifluoromethyl-4H-3,1-benzoxazin-4-one is obtained according to the following:
   1) a step of reacting 3-trifluoromethyl-N-pivaloylaniline with n-butyllithium in an inert solvent and then with carbon dioxide to obtain 2-pivaloylamino-6-trifluoromethylbenzoic acid or a salt thereof,
   2) a step of reacting 2-pivaloylamino-6-trifluoromethylbenzoic acid or a salt thereof with a base in an inert solvent to obtain 2-amino-6-trifluoromethylbenzoic acid or the salt thereof, and
   3) a step of reacting 2-amino-6-trifluoromethylbenzoic acid or the salt thereof with acetic anhydride in an inert solvent.
(9) The production method of (1), wherein 4-[3-(1-pyrrolidinyl)propoxy]aniline or the acid-addition salt thereof is obtained according to the following:
   1) a step of reacting 3-chloro-1-propanol with pyrrolidine in the presence of a base to obtain 3-(1-pyrrolidinyl)-1-propanol,
   2) a step of reacting 3-(1-pyrrolidinyl)-1-propanol with 4-fluoro-1-nitrobenzene in the presence of benzyltriethylammonium chloride to obtain 4-[3-(1-pyrrolidinyl)propoxy]-1-nitrobenzene, and
   3) a step of reducing 4-[3-(1-pyrrolidinyl)propoxy]-1-nitrobenzene in an inert solvent.
(10) 2-Amino-6-trifluoromethylbenzoic acid hydrochloride.
(11) A method for producing 2-amino-6-trifluoromethylbenzoic acid hydrochloride, which comprises reacting 3-trifluoromethyl-N-pivaloylaniline with n-butyllithium in an inert solvent and then with carbon dioxide to obtain 2-pivaloylamino-6-trifluoromethylbenzoic acid or a salt thereof, then reacting the thus-obtained 2-pivaloylamino-6-trifluoromethylbenzoic acid or the salt thereof with a base in an inert solvent to obtain 2-amino-6-trifluoromethylbenzoic acid, and thereafter treating 2-amino-6-trifluoromethylbenzoic acid with hydrochloric acid.
(12) A method for producing 2-amino-6-trifluoromethylbenzoic acid, which comprises reacting 3-trifluoromethyl-N-pivaloylaniline with n-butyllithium in an inert solvent and then with carbon dioxide to obtain 2-pivaloylamino-6-trifluoromethylbenzoic acid or the salt thereof, then reacting the thus-obtained 2-pivaloylamino-6-trifluoromethylbenzoic acid or the salt thereof with a base in an inert solvent.

According to the production method of the invention, 2-methyl-3-{4-[3-(1-pyrrolidinyl)propoxy]phenyl}-5-trifluoromethyl-4(3H)-quinazolinone can be produced safely and efficiently on an industrial scale, which is useful as a preventive or a remedy for metabolic system diseases such as obesity, diabetes, hormone secretion disorder, hyperlipemia, gout, fatty liver; circulatory system diseases such as stenocardia, acute/congestive heart failure, myocardial infarction, coronary arteriosclerosis, hypertension, renal disease, electrolyte disorder; and central and peripheral nervous system diseases such as bulimia, emotional disorder, depression, anxiety, delirium, dementia, schizophrenia, attention deficit hyperactivity disorder, memory impairment, Alzheimer disease, Parkinson disease, sleep disorder, cognitive disorder, motion disorder, paresthesia, dysosmia, epilepsy, morphine resistance, narcotic dependence, alcoholism.

### BEST MODE FOR CARRYING OUT THE INVENTION

The production method for a 4(3H)-quinazolinone derivative of the invention is described concretely.

The step of "reacting 3-trifluoromethyl-N-pivaloylaniline with n-butyllithium in an inert solvent and then with carbon dioxide to produce 2-pivaloylamino-6-trifluoromethylbenzoic acid or a salt thereof " may be carried out as follows: 3-Trifluoromethyl-N-pivaloylaniline is dissolved in an inert solvent such as tetrahydrofuran, and a 1.64 M n-butyllithium/hexane solution is dropwise added to the resulting solution over 1 to 2 hours, while kept at 5°C or lower, and then reacted for 0.5 to 2 hours while still kept at 5°C or lower. Next, an inert solvent such as tetrahydrofuran is added to the resulting reaction liquid, in an amount of 0.5 times of the reaction liquid, and while kept at 5°C or lower, carbon dioxide is introduced into it, and reacted for 1 to 5 hours while still kept at 5°C or lower. The starting material, 3-trifluoromethyl-N-pivaloylaniline in this step may be available according to a known production method, for example, according to the production method described in JP-A-8-104675.

The amount of n-butyllithium to be used is from 2 to 5 mols relative to 1 mol of 3-trifluoromethyl-N-pivaloylaniline.

The amount of carbon dioxide to be used is from 1.5 to 5 mols relative to 1 mol of 3-trifluoromethyl-N-pivaloylaniline.

The step of "reacting 2-pivaloylamino-6-trifluoromethylbenzoic acid or the salt thereof with a base in an inert solvent to produce 2-amino-6-trifluoromethylbenzoic acid" may be carried out as follows: 2-Pivaloylamino-6-trifluoromethylbenzoic acid or the salt thereof is dissolved in an inert solvent such as methyl tert-butyl ether, then concentrated to dryness under reduced pressure, and the resulting residue is dissolved in isopropanol added thereto, and thereafter a base such as sodium hydroxide is gradually added to it, and refluxed for 36 hours to 60 hours.

The amount of the base to be used is from 2 to 5 mols relative to 1 mol of 2-pivaloylamino-6-trifluoromethylbenzoic acid.

The step of "reacting 2-amino-6-trifluoromethylbenzoic acid with hydrochloric acid in an inert solvent to produce 2-amino-6-trifluoromethylbenzoic acid hydrochloride" may be carried out as follows: 2-Amino-6-trifluoromethylbenzoic acid is dissolved in, for example, methanol; then a 4 N hydrochloric acid/ethyl acetate solution is added thereto while kept at 10°C or lower; and thereafter a seed crystal (2-amino-6-trifluoromethylbenzoic acid hydrochloride crystal) is added thereto at 15°C to 25°C, and ripened at 15°C to 25°C for 1.5 to 5 hours; and a 4 N hydrochloric acid/ethyl acetate solution is dropwise added to the resulting suspension over 1 to 2 hours; and ethyl acetate is dropwise added thereto over 3.5 to 5 hours, while kept at 25°C or lower, and ripened overnight at room temperature; and the resulting suspension is cooled to -5°C or lower and ripened for 3 to 5 hours; then the crystal is taken out through filtration, and the resulting crystal cake is washed with methanol and ethyl acetate in that order, and then dried at 30°C to 40°C under reduced pressure.

The amount of the 4 N hydrochloric acid/ethyl acetate solution to be used is from 1.1 mols to 1.5 mols as the amount of hydrochloric acid, relative to 1 mol of 2-amino-6-trifluoromethylbenzoic acid. Preferably, the hydrochloric acid/ethyl acetate solution is, for example, as divided into 2 portions, added in two times.

The amount of ethyl acetate to be used is from 15 to 25 times by weight of 2-amino-6-trifluoromethylbenzoic acid.

The amount of methanol to be used for washing the crystal cake is from 0.2 to 0.5 times by weight of 2-amino-6-trifluoromethylbenzoic acid.

The amount of ethyl acetate to be used for washing the crystal cake is from 3.0 to 5.0 times by weight of 2-amino-6-trifluoromethylbenzoic acid.

The step of "reacting 2-amino-6-trifluoromethylbenzoic acid or the salt thereof with acetic anhydride in an inert solvent to produce 2-methyl-5-trifluoromethyl-4H-3,1-benzoxazin-4-one" may be carried out as follows: 2-Amino-6-trifluoromethylbenzoic acid or the acid-addition salt thereof is suspended in, for example, tetrahydrofuran, then acetic anhydride is added thereto, and refluxed for 5 to 10 hours.

The amount of acetic anhydride to be used is from 4.0 to 10.0 mols relative to 1 mol of 2-amino-6-trifluoromethylbenzoic acid.

The step of "reacting 2-methyl-5-trifluoromethyl-4H-3,1-benzoxazin-4-one with 4-[3-(1-pyrrolidinyl)propoxy]aniline or the acid-addition salt thereof in the presence of an acetic acid-based catalyst to produce 2-methyl-3-{4-[3-(1-pyrrolidinyl)propoxy]phenyl}-5-trifluoromethyl-4(3H)-quinazolinone" may be carried out as follows: Using an acetic acid-based catalyst mentioned below, 2-methyl-5-trifluoromethyl-4H-3,1-benzoxazin-4-one is reacted with 4-[3-(1-pyrrolidinyl)propoxy]aniline or the acid-addition salt thereof at room temperature to 50°C for 10 to 60 hours.

The amount of 4-[3-(1-pyrrolidinyl)propoxy]aniline or the acid-addition salt thereof to be used is from an equimolar amount to 1.5 mols relative to 1 mol of 2-methyl-5-trifluoromethyl-4H-3,1-benzoxazin-4-one.

The acetic acid-based catalyst includes, for example, acetic acid, acetic acid-sodium acetate, or acetic acid-sodium acetate-solvent. In this, the solvent may be, for example, toluene or tetrahydrofuran. Preferred examples of the acetic acid-based catalyst are the following (a) to (d):
(a) Acetic acid of from 10 to 30 times by volume of 2-methyl-5-trifluoromethyl-4H-3,1-benzoxazin-4-one.
(b) Acetic acid of 10 times by volume of 2-methyl-5-trifluoromethyl-4H-3,1-benzoxazin-4-one, and sodium acetate of an equimolar amount to 2-methyl-5-trifluoromethyl-4H-3,1-benzoxazin-4-one.
(c) Acetic acid of 10 times by volume of 2-methyl-5-trifluoromethyl-4H-3,1-benzoxazin-4-one, and tetrahydrofuran of 10 times by volume thereof, and sodium acetate of from an equimolar amount to 5 molar times of 2-methyl-5-trifluoromethyl-4H-3,1-benzoxazin-4-one.
(d) Acetic acid of 10 times by volume of 2-methyl-5-trifluoromethyl-4H-3,1-benzoxazin-4-one, and toluene of 20 times by volume thereof, and sodium acetate of from an equimolar amount to 5 molar times of 2-methyl-5-trifluoromethyl-4H-3, 1-benzoxazin-4-one.

The step of "reacting 3-chloro-1-propanol with pyrrolidine in the presence of a base to produce 3-(1-pyrrolidinyl)-1-propanol" may be carried out as follows. For example, 3-chloro-1-propanol is dissolved in an inert solvent such as toluene, then a base and pyrrolidine are added thereto, and reacted at 50°C to 100°C for 5 to 15 hours.

The amount of 3-chloro-1-propanol, the base and pyrrolidine to be used may be as follows: The base is from 1.2 mols to 3.0 mols, and pyrrolidine is from 1.5 to 3.0 mols, relative to 1 mol of 3-chloro-1-propanol.

The base is, for example, potassium carbonate.

The step of "reacting 3-(1-pyrrolidinyl)-1-propanol with 4-fluoro-1-nitrobenzene in the presence of benzyltriethylammonium chloride to produce 4-[3-(1-pyrrolidinyl)propoxy]-1-nitrobenzene" may be carried out as follows: 3-(1-Pyrrolidinyl)-1-propanol is reacted with 4-fluoro-1-nitrobenzene in an inert solvent such as toluene, in the presence of an aqueous 48 % sodium hydroxide solution and benzyltriethylammonium chloride, at room temperature to 50°C for 10 to 30 hours.

The amount of 3-(1-pyrrolidinyl)-1-propanol, the aqueous 48 % sodium hydroxide solution and benzyltriethylammonium chloride to be used may be as follows: Relative to 1 mol of 3-(1-pyrrolidinyl)-1-propanol, the aqueous 48 % sodium hydroxide solution is from 1.2 to 3.0 mols as sodium hydroxide, and benzyltriethylammonium chloride is from 1.5 mol% to 10.0 mol%.

The step of "reducing 4-[3-(1-pyrrolidinyl)propoxy]-1-nitrobenzene in an inert solvent to product 4-[3-(1-pyrrolidinyl)propoxy]aniline" may be carried out as follows: 4-[3-(1-Pyrrolidinyl)propoxy]-1-nitrobenzene is reacted with formic acid added thereto in an inert solvent such as toluene in the presence of palladium-carbon (Pd-C), at room temperature to 50°C for 10 to 30 hours.

The amount of 4-[3-(1-pyrrolidinyl)propoxy]-1-nitrobenzene, palladium-carbon (Pd-C) and formic acid to be used may be as follows: Palladium carbon (Pd-C) is from 1.5 % by weight to 3.0 % by weight relative to the weight of 4-[3-(1-pyrrolidinyl)propoxy]-1-nitrobenzene, and formic acid is from 2.0 equivalents to 10.0 equivalents relative to 4-[3-(1-pyrrolidinyl)propoxy]-1-nitrobenzene.

In addition, any other reduction method except the above is also applicable to the invention. For example, employable is a method of using hydrogen gas in place of formic acid; or a method using a Raney nickel that is an ordinary catalyst in catalytic hydrogen reduction in place of palladium-carbon. Further, in addition to the reduction method of catalytic hydrogen reduction, also employable herein is any other reduction method of using any other reducing agent of, for example, lithiumaluminium hydride, isobutylaluminium hydride or a combination of metal salts such as sodium borohydride/nickel chloride.

The acid-addition salt of 4-[3-(1-pyrrolidinyl)propoxy]aniline is, for example, p-toluenesulfonate thereof.

The step of converting 4-[3-(1-pyrrolidinyl)propoxy]aniline into the p-toluenesulfonate salt thereof may be carried out as follows: 4-[3-(1-Pyrrolidinyl)propoxy]aniline is dissolved in an inert solvent such as methanol, and p-toluenesulfonic acid monohydrate is added thereto and reacted at room temperature to 30°C for 3 to 5 hours.

The intended compounds produced in the steps as above may be isolated and purified in any known separation and purification method of, for example, concentration, concentration under reduced pressure, solvent extraction, crystallization, reprecipitation, chromatography et al.

### Examples:

The invention is described concretely with reference to the following Examples, to which, however, the invention should not be limited. As the plate in thin-layer chromatography in Examples, used was Silica gel 60F245 (Merck); and for detection, used was a UV detector. As the column silica gel, used was Wakogel^{™} C-300 (Wako Pure Chemical Industries). For electrophoresis (for determination of Cl content), used was Agilent HP³⁰ CE System, and employed was an inorganic ion analysis method. The NMR spectrum was measured, using dimethyl sulfoxide as the internal standard in a heavy dimethyl sulfoxide solution. For this, used were ADVANCE300 (300 MHz; BRUKER) and ADVANCE500 (500 MHz; BRUKER)-type spectrometers; and all δ data were indicated as ppm.

The meanings of the abbreviations in hydrogen nuclear magnetic resonance spectrometry (¹H-NMR) are shown below.
s: singlet,
d: doublet,
dd: double doublet,
t: triplet,
m: multiplet,
br: broad,
q: quartet,
J: coupling constant,
Hz; hertz,
CDCl₃: heavy chloroform,
CD₃OD: heavy methanol,
DMSO-d₆: heavy dimethyl sulfoxide.

### Example 1:

### 1) Production of 3-(1-pyrrolidinyl)-1-propanol:

To a suspension prepared by mixing potassium carbonate (4.8 kg, 34 mol) and toluene (12.3 L), added were pyrrolidine (2.92 kg, 41 mol) and 3-chloro-propanol (2.17 kg, 23 mol), and the mixture was stirred at 85°C for 7 hours. The reaction mixture was cooled to room temperature and filtered, and the solvent of the filtrate was evaporated off under reduced pressure to obtain 3-(1-pyrrolidinyl)-1-propanol/toluene solution (3.34 kg; 3-(1-pyrrolidinyl)-1-propanol/toluene/pyrrolidine = 79/12/2). As a result of quantification through ¹H-NMR, the content of 3-(1-pyrrolidinyl)-1-propanol was 2.8 kg (yield 94 %).

### 2) Production of 4-[3-(1-pyrrolidinyl)propoxy]-1-nitrobenzene:

The toluene solution of 3-(1-pyrrolidinyl)-1-propanol obtained in 1) (content: 2.8 kg) was diluted with toluene (6.6 L) added thereto, and with cooling with ice, aqueous 48 % sodium hydroxide solution (6.6 L) and benzyltriethylammonium chloride (159 g, 0.7 mol) were added to it. Subsequently, 4-fluoronitrobenzene (3.37 kg, 23.8 mol) was dropwise added thereto over 50 minutes, with the inner temperature kept at 60°C or lower. The mixture was stirred overnight, and then water (66 L) was added to it, and extracted twice with methyl t-butyl ether (16.5 L). The extracts were combined, to which was added 1 N hydrochloric acid (33 L) for separation, and the aqueous layer was washed with methyl t-butyl ether (16.5 L). Then aqueous 5 N sodium hydroxide solution (13 L) was added to it, and extracted with methyl t-butyl ether (20 L) to obtain 4-[3-(1-pyrrolidinyl)propoxy]-1-nitrobenzene/methyl t-butyl ether solution (4.71 kg, quantitative yield).

### 3) Production of 4-[3-(1-pyrrolidinyl)propoxy]aniline bis(p-toluenesulfonate):

The 4-[3-(1-pyrrolidinyl)propoxy]-1-nitrobenzene/methyl t-butyl ether solution (4.71 kg) obtained in 2) was evaporated at 40°C under reduced pressure for solvent removal. Methanol (2 L) was added to the residue, and the solvent was again evaporated off. The residue was dissolved in methanol (47.1 L), and degassed twice under reduced pressure, and then 10 % palladium-carbon (with 50 wt.% water) (188 g) was added thereto, and formic acid (2.86 L, 75.2 mol) was added to it over 2.5 hours at room temperature. The mixture was further stirred at room temperature for 2 hours, then filtered, and the filtrate was added to a methanol (14.1 L) solution of p-toluenesulfonic acid (7.88 kg, 41.4 mol). The solution was concentrated at 40°C under reduced pressure to 30 L, and then methyl t-butyl ether (18.8 L) was added thereto, and further a seed crystal (60 g) of 4-[3-(1-pyrrolidinyl)propoxy]aniline bis(p-toluenesulfonate) was added to it. Methyl t-butyl ether (75.4 L) was added to the mixture over 1.5 hours, and stirred overnight at 40°C and the filtered. The solid taken out through filtration was washed with methyl t-butyl ether (18.8 L), and thereafter dried overnight at 40°C under reduced pressure, and then at 50°C for 4 hours to obtain 4-[3-(1-pyrrolidinyl)propoxy]aniline bis(p-toluenesulfonate) (10.6 kg, yield 99.8 %).
¹H-NMR (DMSO-*d*₆, δ ppm): 1.81-1.90 (2H, m), 1.96-2.05 (2H, m), 2.06-2.13 (2H, m), 2.29 (6H, s), 3.02-3.04 (2H, m), 3.28-3.30 (2H, m), 3.57-3.59 (2H, m), 4.05 (2H, t, J=6.1 Hz), 7.03 (2H, d, J=8.8 Hz), 7.12 (4H, d, J=7.8 Hz), 7.28 (2H, d, J=8.8 Hz), 7.49 (4H, d, J=7.8 Hz), 9.49 (1H, brs), 9.73 (2H, brs).

### 4) Production of 2-pivaloylamino-6-trifluoromethylbenzoic acid:

3-Trifluoromethyl-N-pivaloylaniline (3.50 kg, 14.27 mol) was dissolved in tetrahydrofuran (12.5 kg, KF value 138 ppm), then cooled to 5°C or lower, and a 1.64 M n-butyllithium/hexane solution (20.0 L, 32.8 mol) was dropwise added to the mixture over 1 hour or more, while kept at 5°C or lower. Thus obtained, the red suspension was ripened for 1 hour while kept at 5°C or lower. Tetrahydrofuran (6.22 kg) was added to the resulting reaction liquid, and while kept at 5°C or lower, carbon dioxide was introduced into it at a flow rate of 10 L/min over 110 minutes. The completion of the reaction was confirmed by HPLC (high-performance liquid chromatography), and then aqueous 6 % sodium hydrogencarbonate solution (37.7 kg) was added to the reaction liquid, thereafter this was separated into an aqueous layer and an organic solvent layer by liquid-liquid separation. The organic solvent layer was extracted with aqueous 6 % sodium hydrogencarbonate solution (37.7 kg). The obtained aqueous layers were combined, adjusted to have a pH of 3.0 or less by adding 6 N hydrochloric acid thereto, and then extracted twice with methyl t-butyl ether (hereinafter abbreviated as "MTBE") (25.9 kg). The obtained MTBE layers were combined, and washed with 20 % saturated saline (18 kg) to obtain an MTBE solution containing 2-pivaloylamino-6-trifluoromethylbenzoic acid (2.94 kg (found), yield; 71 %).

### 5) Production of 2-amino-6-trifluoromethylbenzoic acid hydrochloride:

The MTBE solution contianing 2-pivaloylamino-6-trifluoromethylbenzoic acid (5.87 kg (found), 20.3 mol) obtained in 4) was concentrated under reduced pressure, and the resulting residue was dissolved in isopropanol (69.8 kg). Sodium hydroxide (4.58 kg, 114.17 mol) was gradually added to the solution, and refluxed (83.1°C) for 48 hours. HPLC confirmed the completion of the reaction, and the resulting reaction liquid was cooled to 30.5°C; then water (35 L) was added thereto, and further this was adjusted to have a pH of from 1 to 3 by adding 3 N hydrochloric acid (44.4 kg) thereto, and extracted twice with isopropyl acetate (30.6 kg). The isopropyl acetate layers were combined, washed with 20 % ammonium chloride (75.5 kg), and concentrated under reduced pressure. The resulting residue (containing 4.03 kg (found, 19.6 mol) of 2-amino-6-trifluoromethylbenzoic acid) was dissolved in methanol (30.7 kg), and cooled to 10°C. To the resulting solution, added was a 4 N hydrochloric acid/ethyl acetate solution (2.77 kg, 11.9 mol), and then a seed crystal (2-amino-6-trifluoromethylbenzoic acid hydrochloride crystal, 27.1 g) was added to it, and ripened at 15°C for 1.5 hours. To the resulting suspension, dropwise added was a 4 N hydrochloric acid/ethyl acetate solution (2.56 kg, 11.6 mol), over 1 hour or more, while kept at 25°C or lower, and then ethyl acetate (87.5 kg) was dropwise added thereto over 3.5 hours or more. The resulting suspension was ripened overnight at room temperature, and then cooled to 5°C or lower, over 2 hours or more, and thereafter ripened for 3 hours. Further, the resulting suspension was filtered, and the crystal cake was washed repeatedly twice with a mixture of methanol (1.38 kg) and ethyl acetate (15.9 kg), and dried overnight at 40°C under reduced pressure to obtain 2-amino-6-trifluoromethylbenzoic acid hydrochloride (4.48 kg, purity 96.4 %, yield 63 %) as a colorless needle crystal.
m.p.: 330°C (decomposition point).
Electrophoresis: Cl content, 0.95 mol.
¹H-NMR (500 MHz, DMSO-d₆, δ ppm): 6.97 (1H, d, J=7.4 ppm), 7.09 (1H, d, J=8.1 ppm), 7.33 (1H, dd, J=7.4, 8.1 ppm), 8.88 (4H, br).
HPLC condition:
Column: YMC Pack ODS-AM, AM303,
Temperature: 40°C,
Mobile phase: 0.1 % pH 7.0 phosphate buffer/n-propylamine-acetonitrile (gradient),
0 min: 95/5,
15 min: 65/35,
20 min: 50/50,
28 min: 20/80,
30 min: 20/80,
Flow rate: 1.0 mL/min,
Detection UV wavelength: 220 nm.

### 6) Production of 2-methyl-5-trifluoromethyl-4H-3, 1-benzoxazin-4-one:

Acetic anhydride (23.5 mL, 249 mmol) was added to a THF (120 mL) suspension of 2-amino-6-trifluoromethylbenzoic acid hydrochloride (12.0 g (96 wt.%), 47.7 mmol), and the mixture was heated under reflux for 6 hours. The mixture was cooled to room temperature, then filtered, and the filtrate was concentrated under reduced pressure to 24 mL. N-heptane (120 mL) was added to the residue over 30 minutes at 45 to 50°C, and the mixture was cooled to room temperature over 1 hour, and then further stirred for 15 hours. The formed crystal was taken out through filtration, then washed with n-heptane (24 mL), and dried under reduced pressure at 60°C for 15 hours to obtain the entitled compound as a pale yellow solid (9.80 g, yield 90 %).

### 7) Production of 2-methyl-3-{4-[3-(1-pyrrolidinyl)propoxy]phenyl}-5-trifluoromethyl-4(3H)-quinazolinone:

Toluene (41.4 kg), 2-methyl-5-trifluoromethyl-4H-3,1-benzoxazin-4-one (3.00 kg, 13.1 mol), 4-[3-(1-pyrrolidinyl)propoxy]aniline bis(p-toluenesulfonate) (7.40 kg, 13.1 mol), sodium acetate (2.26 kg, 27.6 mol) and acetic acid (7.90 kg, 132 mol) were mixed, and stirred at 30°C for 50 hours. The reaction mixture was cooled to 20°C or lower, then water (24 L) and aqueous 5 N sodium hydroxide solution (36 L) were added thereto in that order, and the organic layer was separated. The aqueous layer was extracted with toluene (30 L), and the organic layers were combined and washed with aqueous 1 N sodium hydroxide solution (30 L) and water (30 L) in that order. This washing was further repeated twice in the same manner as previously, and then washed with aqueous 20 % sodium chloride solution (30 L). Using toluene, the organic layer was azeotropically dewatered at 29 to 46°C under reduced pressure, with keeping its capacity, 60 L as such (KF: 112.8 ppm), and then this was treated with activated charcoal (Shirasagi P (301 g)) added thereto for 1.25 hours at room temperature. The activated charcoal was removed through filtration, the residue was washed twice with toluene (15 L), then the filtrate and the wash liquid were combined, and concentrated under reduced pressure to about 18 L. The residue was heated at about 50°C, then n-heptane (8.3 kg) and a seed crystal of 2-methyl-3-{4-[3-(1-pyrrolidinyl)propoxy]phenyl}-5-trifluoromethyl-4(3H)-quinazolinone (300 g) were added to it in that order, and stirred at 50°C for 1 hour. Then n-heptane (86.25 kg) was added to it over 3 hours at 50°C. The resulting suspension was cooled to room temperature, stirred overnight, then further cooled to 0°C and stirred for 4.8 hours. The formed crystal was taken out through filtration, washed twice with n-heptane (15 L), and dried overnight at 40°C under reduced pressure to obtain the entitled compound as a crude product of a colorless crystalline solid (4.84 kg, yield 86 %). This was dissolved in N,N-dimethylacetamide (46.9 L), warmed to 50°C, and then water (16.52 L) and a seed crystal of 2-methyl-3-{4-[3-(1-pyrrolidinyl)propoxy]phenyl}-5-trifluoromethyl-4(3H)-quinazolinone (47 g) were added to it in that order at 50°C, and then stirred at 50°C for 1 hour. Further, water (30.58 L) was added to it over 2.8 hours at 50°C, and then cooled to room temperature, and stirred overnight. The formed crystal was taken out through filtration, washed twice with water (23.5 L), and then dried overnight at 40°C under reduced pressure to obtain the entitled compound as a colorless crystalline solid (4.14 kg, yield 88 %).
¹HNMR (400 MHz, CDCl₃, δ ppm): 1.77-1.83 (4H, m), 2.00-2.08 (2H, m), 2.26 (3H, s), 2.51 2.57 (4H, m), 2.63 (2H, t, J=7.2 Hz), 4.07 (2H, t, J=6.8 Hz), 7.03 (2H, d, J=8.8 Hz), 7.12 (2H, d, J=8.8 Hz), 7.79 (1H, t, J=7.6 Hz), 7.82-7.88 (2H, m)

### Examples 2 to 7:

Using the acetic acid-based catalyst shown in Table 1 and using the same starting compound as in Example 1-7) under the same condition as therein, 2-methyl-3-{4-[3-(1-pyrrolidinyl)propoxy]phenyl}-5-trifluoromethyl-4(3H)-quinazolinone was produced, and the yield is shown in Table 1.

**Table 1**

| Example No. | Acetic Acid-Based Catalyst | | | Yield (%) |
|---|---|---|---|---|
| | amount of acetic acid | amount of sodium acetate (equivalent) | solvent (amount) | |
| 2 | 10 | not used | not used | 80 |
| 3 | 20 | not used | not used | 76 |
| 4 | 10 | 1 | not used | 80 |
| 5 | 10 | 1 | THF(10) | 88 |
| 6 | 10 | 2 | THF(10) | 93 |
| 7 | 4 | not used | not used | 55 |

The amount of acetic acid and the solvent was shown by volume relative to one volume of 5-trifluoromethyl-4-oxo-2-methylbenzoxazine.

From Table 1, it is obvious that when a large amount of acetic acid is used or when sodium acetate and/or tetrahydrofuran as a solvent is added, then the yield of 2-methyl-3-{4-[3-(1-pyrrolidinyl)propoxy]phenyl}-5-trifluoromethyl-4(3H)-quinazolinone is increased.

### INDUSTRIAL APPLICABILITY

The invention provides an excellent industrial-scale production method for 2-methyl-3- {4-[3-(1-pyrrolidinyl)propoxy]phenyl}-5-trifluoromethyl-4(3H)-quinazolinone which is useful as a preventive or a remedy for metabolic system diseases such as obesity, diabetes, hormone secretion disorder, hyperlipemia, gout, fatty liver; circulatory system diseases such as stenocardia, acute/congestive heart failure, myocardial infarction, coronary arteriosclerosis, hypertension, renal disease, electrolyte disorder; and central and peripheral nervous system diseases such as bulimia, emotional disorder, depression, anxiety, delirium, dementia, schizophrenia, attention deficit hyperactivity disorder, memory impairment, Alzheimer disease, Parkinson disease, sleep disorder, cognitive disorder, motion disorder, paresthesia, dysosmia, epilepsy, morphine resistance, narcotic dependence, alcoholism.

## Claims

1. A method for producing 2-methyl-3-{4-[3-(1-pyrrolidinyl)propoxy]phenyl}-5-trifluoromethyl-4(3H)-quinazolinone, which comprises reacting 2-methyl-5-trifluoromethyl-4H-3,1-benzoxazin-4-one with 4-[3-(1-pyrrolidinyl)propoxy]aniline or an acid-addition salt thereof in the presence of an acetic acid-based catalyst.

2. The production method as claimed in claim 1, wherein the acid-addition salt of 4-[3-(1-pyrrolidinyl)propoxy]aniline is 4-[3-(1-pyrrolidinyl)propoxy]aniline bis(p-toluenesulfonate).

3. The production method as claimed in claim 1, wherein the acetic acid-based catalyst is acetic acid.

4. The production method as claimed in claim 3, wherein the amount of acetic acid is from 10 to 30 times by volume of 4-[3-(1-pyrrolidinyl)propoxy]aniline or the acid-addition salt thereof.

5. The production method as claimed in claim 1, wherein the acetic acid-based catalyst is acetic acid and sodium acetate.

6. The production method as claimed in claim 1, wherein the acetic acid-based catalyst is acetic acid, sodium acetate and solvent.

7. The production method as claimed in claim 6, wherein the solvent is toluene or tetrahydrofuran.

8. The production method as claimed in claim 1, wherein 2-methyl-5-trifluoromethyl-4H-3,1-benzoxazin-4-one is obtained according to the following:
1) a step of reacting 3-trifluoromethyl-N-pivaloylaniline with n-butyllithium in an inert solvent and then with carbon dioxide to obtain 2-pivaloylamino-6-trifluoromethylbenzoic acid or a salt thereof,
2) a step of reacting 2-pivaloylamino-6-trifluoromethylbenzoic acid or the salt thereof with a base in an inert solvent to obtain 2-amino-6-trifluoromethylbenzoic acid or a salt thereof, and
3) a step of reacting 2-amino-6-trifluoromethylbenzoic acid or the salt thereof with acetic anhydride in an inert solvent.

9. The production method as claimed in claim 1, wherein 4-[3-(1-pyrrolidinyl)propoxy]aniline or the acid-addition salt thereof is obtained according to the following:
1) a step of reacting 3-chloro-1-propanol with pyrrolidine in the presence of a base to obtain 3-(1-pyrrolidinyl)-1-propanol,
2) a step of reacting 3-(1-pyrrolidinyl)-1-propanol with 4-fluoro-1-nitrobenzene in the presence of benzyltriethylammonium chloride to obtain 4-[3-(1-pyrrolidinyl)propoxy]-1-nitrobenzene, and
3) a step of reducing 4-[3-(1-pyrrolidinyl)propoxy]-1-nitrobenzene in an inert solvent.
